# EUROPÄISCHE PATENTSCHRIFT

(11) **EP 0 842 424 B1**
(45) Veröffentlichungstag und Bekanntmachung des Hinweises auf die Patenterteilung: **03.11.1999**
(21) Anmeldenummer: 96924736.0
(22) Anmeldetag: 12.07.1996
(51) Int. Cl.: G01N 33/50

(54) **VERFAHREN ZUR BESTIMMUNG DER WIRKSAMKEIT UND VERTRÄGLICHKEIT EINES XENOGENEN STOFFES AUF EINEN ORGANISMUS**
PROCESS FOR DETERMINING THE EFFECTIVENESS AND TOLERANCE OF A XENOGENIC SUBSTANCE ADMINISTERED TO AN ORGANISM
PROCEDE POUR DETERMINER L'EFFICACITE ET LA TOLERANCE D'UNE SUBSTANCE XENOGENIQUE ADMINISTREE A UN ORGANISME

(30) Priorität: 12.07.1995 DE 19525133
(43) Veröffentlichungstag der Anmeldung: 20.05.1998
(73) Patentinhaber: Lauk, Christiane, 71159 Mötzingen (DE)
(72) Erfinder: Lauk, Christiane, 71159 Mötzingen (DE)
(74) Vertreter: Schuster, Gregor, Dipl.-Ing.
(86) Internationale Anmeldenummer: DE9601269
(87) Internationale Veröffentlichungsnummer: WO9703356

(56) Entgegenhaltungen:
- JOURNAL OF IMMUNOLOGICAL METHODS, Bd. 142, 1991, AMSTERDAM, Seiten 257-265, XP002018218 ROEHM ET AL.: "An improved colorimetric assay for cell proliferation ...."
- NATURWISSENSCHAFTEN, Bd. 76, Nr. 11, 1.November 1989, BERLIN, Seiten 530-531, XP000196390 HUELSEN ET AL: "INFLUENCE OF VISCUM ALBUM PREPARATIONS ON THE NATURAL KILLER CELL-MEDIATED CYTOTOXICITY OF PERIPHERAL BLOOD" in der Anmeldung erwähnt
- BLOOD, Bd. 84, Nr. 10, 15.November 1994, PHILADELPHIA, PA, Seiten 3440-3446, XP000196389 SILBER ET AL: "CHEMOSENSITIVITY OF LYMPHOCYTES FROM PATIENTS WITH B-CELL CHRONIC LYMPHOCYTIC LEUKEMIA TO CHLORAMBUCIL, FLUDARABINE, AND CAMPTOTHECIN ANALOGS" in der Anmeldung erwähnt
- PATENT ABSTRACTS OF JAPAN vol. 018, no. 226 (C-1194), 25.April 1994 & JP,A,06 022799 (NIPPON SUISAN KAISHA LTD), 1.Februar 1994,

## Beschreibung

### Stand der Technik

Die Erfindung geht aus von einem Verfahren zur Bestimmung der Aktivität von Immunzellen in Abhängigkeit von einem Wirkstoffes nach der Gattung des Hauptanspruchs.

Die Bestimmung einerseits der Verträglichkeit und andererseits der Wirksamkeit von Arzneimitteln, wie beispielsweise von homöopathischen, biologischen, natürlichen und chemischen Stoffen und Stoffgemischen auf den zu behandelnden Patienten ist für eine erfolgreiche Therapie und Behandlung einer Krankheit entscheidend. Normalerweise wird nach Verabreichen eines Arzneimittels die Reaktion des Patienten auf das Arzneimittel untersucht.

Bekannt sind verschiedene Verfahren, bei denen die Aktivität von Immunzellen gegenüber verschiedenen Zellen untersucht werden.

Bei einem bekannten Verfahren (In: Naturwissenschaften 76, S. 530 ff., 1989) wird der Einfluß der Mistelpflanze (Viscum album) auf die Aktivität von Immunzellen gegenüber Krebszellen untersucht. Hierzu werden Krebszellen mit radioaktivem Tritium markiert und mit Immunzellen und Mistelextrakt vermischt. Nach einer gewissen Zeit wird die Radioaktivität des Gemischs im Überstand bestimmt, die einen Aufschluß darüber gibt, wieviele Krebszellen von den Immunzellen vernichtet worden sind. Der Nachteil dieses Verfahrens besteht darin, daß radioaktive Isotope benötigt werden, daß die Genauigkeit von der Anzahl der untersuchten Zellen abhängt und daß die Durchführung des Verfahrens aufwendig und zeitintensiv ist.

Bei einer anderen bekannten Untersuchung (In: Blood, Vol, 84, No. 10, November 1994, S. 3440 - 3346) wird die Chemosensitivität von B-Lymphozyten unter Verwendung des MTT-Analyseverfahrens bestimmt. Hierbei wird die Cytotoxizität von Chlorambozil (CLB) verglichen mit derjenigen von Fludarabine, von DNA Topoisomerase 1 Inhibitoren u. ä. Stoffen. Die untersuchten B-Lymphozyten stammen hierbei von Patienten, die unter B-CLL (chronic lymphocytic leukemia) leiden. Das Ziel dieser Untersuchung ist es, eine allgemeine Aussage über die Wirkung der verwendeten und untersuchten Stoffe bezogen auf eine unter einer speziellen Krankheit leidenden Gruppe von Patienten zu machen.

Bei dem gattungsgemäßen bekannten Verfahren (In: Cancer Immunology Immunotherapy, S. 393-398, 1992) wird der Einfluß von körpereigenen Stoffen (Interferon γ, Tumor-Nectrosis-Faktor-α) auf Cytostatika und Cytotoxizität bei menschliche Gewebezellen (U 937) untersucht. Bei dieser Untersuchung wird versucht eine allgemeine Aussage über die Wirkung der untersuchten Stoffe bezogen auf eine spezielle Art von Gewebezellen zu machen. Es geht darum, eine gewisse zu erwartende Wirkung zu überprüfen.

### Die Erfindung und ihre Vorteile

Demgegenüber hat das erfindungsgemäße Verfahren mit den kennzeichnenden Merkmalen des Hauptanspruchs den Vorteil, daß die Verträglichkeit und Wirksamkeit xenogener Arzneimittel auf eine menschliche Person oder ein Einzeltier bestimmt werden kann. Hierzu wird das gattungsgemäße, wissenschaftlich untersuchte Verfahren, erfindungsgemäß für einen neuen, gewerblich anwendbaren Zweck eingesetzt.

Das Ziel der gattungsgemäßen Untersuchung ist Wirkungen körpereigener Stoffe auf Immunzellen und Targetzellen, welche infizierte Zellen, Krebszellen oder andere Zellen sein können, zu ermitteln. In erster Linie sollte hierbei eine allgemeine Aussage getroffen werden, ob ein bestimmter Stoff in irgendeiner Beziehung zu Immunzellen und Immunsystem von bestimmten Lebewesen steht.

Die erfindungsgemäße neue Anwendung des modifizierten Verfahrens hingegen ist nicht dazu bestimmt eine allgemeingültige Aussage über die Wirkung bestimmter körpereigener Stoffe zu machen, sondern es wird die individuelle Verträglichkeit und Wirksamkeit eines körperfremden Arzneimittels auf einen Patienten bestimmt. Das Ergebnis des Verfahrens gibt Aufschluß über einen möglichen Behandlungserfolg. So kann vor dem Verabreichen eines Arzneimittels mit Hilfe des erfindungsgemäßen Verfahrens ermittelt werden, ob und wie der einzelne Patient auf das Arzneimittel reagiert. Es können auch mehrere vergleichbare Präparate getestet werden, um dasjenige zu ermitteln, auf welches der Patient am meisten anspricht. Für derartige Untersuchungen wird dem Patienten Blut abgenommen, aus dem die Immunzellen isoliert werden. Diese Zellen werden mit speziellen Targetzellen beispielsweise mit virusinfizierten Zellen, mit Krebszellen oder mit normalen Zellen (autogene, allogene oder xenogene Zellen) und mit dem Arzneimittel versetzt.

Ohne Zweifel ist es ein seit langem anhaltendes Bedürfnis, auf einfache und kostengünstige Art und Weise vor dem Verabreichen eines Arzneimittels bestimmen und vorhersagen zu können, ob und wie der Patient auf das Arzneimittel reagiert bzw. ob ein möglicher Behandlungserfolg eintritt. Mit Hilfe des erfindungsgemäßen Verfahrens wird dieses Bedürfnis befriedigt.

Obwohl das gattungsgemäße Verfahren schon mindestens seit 1992 und die zur Durchführung des Verfahrens auf dem Tetrazoliumsalz MTT beruhende Aktivitätsanalyse für ähnliche Zwecke schon seit mindestens 1988 (vgl. Cancer Resaerch 48: 589-601, 1988) für wissenschaftliche Untersuchungen bekannt ist, wurde bis zu dem Zeitrang der Erfindung das gattungsgemäße Verfahren nur auf dem Gebiet der wissenschaftlichen Analytik benutzt.

Ein weiterer Vorteil des erfindungsgemäßen Verfahrens ist, daß sehr teure und/oder mit Risiken und Nebenwirkungen behaftete Arzneimittel auf die Wirksamkeit und Verträglichkeit mit dem Patienten vor Verabreichen getestet werden können. Oftmals ist gerade bei solchen Mitteln ein Wirksamkeitsnachweis des Arzneimittels zur Kostenerstattung der Arzneimittelkosten durch die Krankenkasse/Versicherung zwingend erforderlich.

Ein weiterer Vorteil der Erfindung ist, daß das Verfahren zeitgleich oder bei nicht erwünschten Wirkungen nacheinander mit verschiedenen xenogenen Arzneimitteln und/oder Arzneimittelmischungen durchgeführt werden kann. Ein solches Vorgehen weist den Vorteil auf, daß die optimale Wirksamkeit und Verträglichkeit möglicher alternativ zur Auswahl stehender xenogener Arzneimittel bestimmt werden kann.

Nach einer vorteilhaften Ausgestaltung der Erfindung werden als xenogene Arzneimittel homöopathische Wirkstoffe und Wirkstoffgemische verwendet. Insbesondere in der Homöopathie, in der zur Behandlung von Krankheiten sehr geringe Dosen von Wirkstoffen verabreicht werden, die in höheren Dosen beim Gesunden ein ähnliches Krankheitsbild hervorrufen, ist es sinnvoll Wirkung und Verträglichkeit der Präparate vor Verabreichen an den Patienten zu bestimmen. Zudem weist das Verfahren den Vorteil auf, daß die Wirksamkeit und Verträglichkeit an höheren Dosen getestet werden kann, als dies am Patienten der Fall ist.

Nach einer weiteren vorteilhaften Ausgestaltung der Erfindung werden als Targetzellen Krebszellen verwendet. So kann insbesondere bei Krebspatienten der Einfluß eines Arzneimittels untersucht werden.

Nach einer weiteren vorteilhaften Ausgestaltung der Erfindung werden als Targetzellen virusinfizierte Zellen verwendet.

Nach einer weiteren vorteilhaften Ausgestaltung der Erfindung werden als Targetzellen normale Zellen (allogene, autogene oder xenogene Zellen) verwendet.

Nach einer weiteren vorteilhaften Ausgestaltung der Erfindung werden als Farbstoffe Tetrazoliumsalze verwendet.

Nach einer weiteren vorteilhaften Ausgestaltung der Erfindung wird als Substrat das Tetrazoliumsalz MTT verwendet. Bei diesem Substrat handelt es sich um ein gelbes Tetrazoliumsalz. Durch die Dehydrogenase in den aktiven Mitochondrien lebender Zellen wird dieses gelbe Salz in blaue Formazankristalle umgewandelt. Diese Umwandlung vollzieht sich nur in lebenden Zellen und ist bei Immunzellen und Targetzellen unterschiedlich stark. Mit Hilfe eines Spektrometers wird die Farbe der Probe ermittelt, welche wiederum Aufschluß darüber gibt, wieviele Targetzellen durch die Immunzellen vernichtet worden sind.

Nach einer weiteren vorteilhaften Ausgestaltung der Erfindung wird als Substrat das Tetrazoliumsalz XTT verwendet. Dieses Substrat weist bei speziellen Anwendungen genauere Meßergebnisse auf.

Weitere Vorteile und vorteilhafte Ausgestaltungen der Erfindung sind den Ansprüchen entnehmbar.

Alle in der Beschreibung und den nachfolgenden Ansprüchen dargestellten Merkmale können sowohl einzeln als auch in beliebiger Kombination miteinander erfindungswesentlich sein.

## Patentansprüche

1. Verfahren zur Bestimmung der Aktivität von Immunzellen in Abhängigkeit von einem Wirkstoff mit folgenden Verfahrensschritten:
- Isolieren von Immunzellen,
- Zugabe von Targetzellen,
- Zugabe eines Substrats, welches durch die Aktivität von Zellen seine Struktur ändert,
- Bestimmen der Grundaktivität des Gemisches aus Immunzellen, Targetzellen und Substrat mittels spektrometrischer Analyse,
- Zugabe des Wirkstoffes
- Messen der Reaktionsaktivität des Gemisches mittels spektrometrischer Analyse,
- Vergleich der Meßergebnisse der Grundaktivität und der Reaktionsaktivität des Gemisches
- Bestimmen der Reaktionsstärke aufgrund des Vergleichs,
dadurch gekennzeichnet,
- daß als Wirkstoff xenogene (körperfremde) Arzneimittel verwendet werden,
- daß als Immunzellen die Immunzellen nur einer menschlichen Person oder eines Einzeltieres verwendet werden,
- daß die Reaktion der Immunzellen auf das xenogene Arzneimittel für den Organismus individuell ausgewertet wird,
- daß aus der Auswertung die Verträglichkeit und/oder Wirksamkeit des xenogenen Arzneimittels auf den Organismus bestimmt wird und
- daß erforderlichenfalls das Verfahren zeitgleich oder bei nicht erwünschten Wirkungen nacheinander mit verschiedenen xenogenen Arzneimitteln und/oder Arzneimittelmischungen durchgeführt wird, um die optimale Wirksamkeit und Verträglichkeit möglicher alternativ zur Auswahl stehender xenogener Arzneimittel zu bestimmen.

2. Verfahren nach Anspruch 1, dadurch gekennzeichnet, daß als xenogene Arzneimittel homöopathische Wirkstoffe, Naturstoffprodukte pflanzlichen, tierischen und bakteriellen Ursprungs sowie Wirkstoffgemische verwendet werden.

3. Verfahren nach Anspruch 1, dadurch gekennzeichnet, daß als Targetzellen Krebszellen verwendet werden.

4. Verfahren nach Anspruch 1, dadurch gekennzeichnet, daß als Targetzellen virusinfizierte Zellen verwendet werden.

5. Verfahren nach Anspruch 1, dadurch gekennzeichnet, daß als Targetzellen normale Zellen (allogene, autogene oder xenogene Zellen) verwendet werden.

6. Verfahren nach einem der vorhergehenden Ansprüche, dadurch gekennzeichnet, daß als Substrat ein Tetrazoliumsalz verwendet wird.

7. Verfahren nach Anspruch 6, dadurch gekennzeichnet, daß das Tetrazoliumsalz MTT (3-{4,5 dimenthylthiazol-2-yl}-2,5-diphenyl tetrazolium bromid) verwendet wird.

8. Verfahren nach Anspruch 6, dadurch gekennzeichnet, daß das Tetrazoliumsalz XTT (2,3 bis{2-methoxy-4-nitro-5-sulfophenyl}-5-({phenylamino}carbonyl)-2H-terazolyum hydroxid) verwendet wird.

## Revendications

1. Procédé pour déterminer l'efficacité des cellules immunisés dépendant d'une étoffe avec les pas de procédure suivants:
- Isolation des cellules immunisées,
- supplément des target cellules,
- supplément d'un substrat, qui change sa structure par l'activité des cellules,
- détermination de l'activité de base du melange des cellules immunisées, target et substrat par moyen d'une analyse spectrométrique,
- supplément de la substance,
- mesurer de l'activité réactionnelle du mélange par l'analyse spectrométrique,
- comparaison des valeurs mésurées de l'activité de fond et de l'activité de la réaction du mélange,
- determination de la force réactionnaire en base de la comparaison,
caractérisé du fait
- que des remèdes xenogenétiques (hétérogène du corps) sont utilisés comme substance,
- que les cellules immunisées d'une seule personne ou d'un animal sont utilisées comme cellules immunisées,
- que la réaction des cellules immunisées envers le remède xénogène sera évaluée individuellement pour l'organisme,
- que le reconciliant hors de l'évaluation et/ou efficacité du remède xénogène sera déterminé sur l'organisme,
- que le procès, si nécéssaire, aura lieu simultanément ou si les efficacités ne seront pas désirées, l'un après l'autre avec de différents remèdes xénogènes et/ou des mélanges de remèdes seront exécutés pour déterminer l'efficacité optimale et le reconciliant des remèdes xénogènes alternativement à disposition,

2. Procédé selon titre 1, caractérisé du fait que comme remèdes xénogènes sont utilisés des substances homéopatiques, produits de substances naturelles, d'origine animale et bactérielle ainsi que des mélanges.

3. Procédé selon titre 1, caractérisé du fait que des cellules target sont utilisés commes cellules cancer.

4. Procédé selon titre 1, caractérisé du fait que des cellules infisciés des vires sont utilisés commes cellules target.

5. Procédé selon titre 1, caractérisé du fait que des cellules normales (allogènes, autogènes ou xénogènes) sont utilisées comme cellules target.

6. Procédé selon l'un des titres précédents, caractérisés du fait que un sel tétrazolium est utilisé comme substrat.

7. Procédé selon tittre 6, caratérisé du fait que le sel tetrazolium MTT (3- 4,5 dimenthylthiazol 2 yl 2,5 diphenyl tetrazolium bromid ) est utilisé.

8. Procédé selon titre 6, caractérisé du fait que le sel tetrazolium XTT (2,3 à 2 methoxy-4-nitro-5-sulfophenyl 5 phenylamino carbonyl 2H terazolyum hydroxid)est utilisé.

## Claims

1. Process for determining the activity of immune cells in dependence of substance effectiveness by means of the following proceeding steps:
- isolation of immune cells,
- addition of a substrat changing its structure by activity of cells,
- determination of the basic activity of the mix of immune cells, target cells and substrat by means of spectrometric analysis,
- addition of the effective substance,
- measurement of the reaction activity of the mix by means of spectrometric analysis,
- comparison of the measuring results of the basic activity and of the reaction activity of the mix,
- determination of the reaction effectivity on the basis of a comparison,
caracterized by the fact
- that as substance will be used xogenic remedies(heterogene to the body),
- that as immune cells can be used the immune cells of only one person or one animal,
- that the reaction of the immune cells to the xenogenic remedy for the organism will be evaluated individually,
- that compatibility and/or effectivity of xenogenic remedy on the organism is determinated by the evaluation,
- that if required, the process will take place simultaneously or if effectivity is not desired, after one another with different xenogenic remedy, and/or remedy mix, in order to determine optimum efficacity and compatibility of alternative xenogenic remedy.

2. Process according to Title 1, caracterized as far as there will be used as xenogetic remedy homeopatic substances, natural substance products of plants, animal and bacteriel sources as well as sub stance mix.

3. Process according to Title 1, caracterized as far as cancer cells will be used as target cells.

4. Process according to Title 1, caracterized as far as virus infec ted cells are used as target cells.

5. Process according to Title 1, caracterized s far as normal cells (allogenetic, autogene or xenogenetic cells) be used as target cells.

6. Process according to one of the preceeding Titles, caracterized as far as is used tetracolium salt as substrat.

7. Process according to Title 6, caracterized as far as the tetrazolium salt MTT (3- 4,5 dimenthylthiazol-2-yl - 2,5-diphenyl tetrazolium bromid) is used.

8. Process according to Title 6, caracterized as far as tetrazolium salt XTT (2,3 to 2-methoxy-4-nitro-5-sulfophenyl-5- phenylamino carbonyl)-2H-terazolyum hydroxid) is used.
